(19) Europäisches Patentamt
European Patent Office
Office européen des brevets

(11) Publication number: **0 068 664**
**B1**

(12) **EUROPEAN PATENT SPECIFICATION**

(45) Date of publication of patent specification: **14.05.86**

(51) Int. Cl.$^4$: **H 01 M 4/90**, H 01 M 8/16, C 25 B 11/04, C 12 M 1/40

(21) Application number: **82302910.3**

(22) Date of filing: **07.06.82**

(54) **A heme protein immobilized electrode.**

(30) Priority: **12.06.81 JP 89615/81**

(43) Date of publication of application:
**05.01.83 Bulletin 83/01**

(45) Publication of the grant of the patent:
**14.05.86 Bulletin 86/20**

(84) Designated Contracting States:
**DE FR GB**

(56) References cited:
**WO-A-80/00453**
**DE-A-2 049 008**
**FR-A-1 303 200**
**FR-A-1 361 241**
**FR-A-2 495 843**
**US-A-3 226 262**
**US-A-3 403 081**

**CHEMICAL ABSTRACTS, vol. 91, no. 20, November 1979, page 520, no. 165439s, Columbus Ohio (USA); H. JAHNKE et al.: "Cathodic reduction of oxygen on chelates"**

(73) Proprietor: **AJINOMOTO CO., INC.**
**5-8, Kyobashi 1-chome, Chuo-ku**
**Tokyo 104 (JP)**

(72) Inventor: **Niki, katsumi**
**No. 243, Mamedo-cho Kouhoku-ku**
**Yokohama-shi Kanagawa-ken (JP)**
Inventor: **Yagi, Tatsuhiko**
**No. 1664-33, Kita**
**Shizuoka-shi Shizuoka-ken (JP)**
Inventor: **Inokuchi, Hiroo**
**No. 39, Nishigounaka Myodaijimachi**
**Okazaki-shi Aichi-ken (JP)**
Inventor: **Nakamura, Asao**
**No. 3-23-15, Nakazato**
**Kita-ku Tokyo (JP)**

(74) Representative: **Bond, Bentley George et al**
**HASELTINE LAKE & CO. Hazlitt House 28**
**Southampton Buildings Chancery Lane**
**London, WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 664 B1

(56) References cited:
CHEMICAL ABSTRACTS, vol. 84, no. 2, 12th January 1976, page 374, no. 10430p, Columbus Ohio (USA); V.A. BOGDANOVSKAYA et al.: "Electrocatalysis for organic complexes. I. Electroreduction of oxygen in the presence of iron-containing proteins and their active groups"

CHEMICAL ABSTRACTS, vol. 83, no. 21, 24th November 1975, page 204, no. 174420r, Columbus Ohio (USA); YAGI, TATSUHIKO et al.: "New assay method for hydrogenase based on an enzymic electrode reaction. Enzymic electric cell method"

CHEMICAL ABSTRACTS, vol. 86, no. 4, 24th January 1977, page 178, no. 19468f, Columbus Ohio (USA); YAGI, TATSUHIKO et al.: "Separation of hydrogenase-catalyzed hydrogen-evolution system from electron-donating system by means of enzymic electric cell technique"

**Description**

The invention relates to an electrode whose surface is activated by an immobilized layer of a heme protein, the electrode having an electrocatalytic activity for the direct four-electron reduction of dioxygen and an enzymatic activity.

During the last five years, there has been a great deal of interest in the manufacture of chemically modified electrodes or catalytic electrodes. The manufacture of oxygen electrodes catalyzing the electrochemical reduction of dioxygen in accordance with the following two reactions:

(I) (a) $O_2+2e+2H^+\rightarrow H_2O_2$
(b) $H_2O_2+2e+2H^+\rightarrow 2H_2O$
(II) $O_2+4e+4H^+\rightarrow 2H_2O$

is one of the most important subjects in the development of fuel cells.

It is known to use electrodes on which either heme protein or laccase is immobilized to catalyze the electrochemical reduction of dioxygen (M. R. Tarasevich, Bioelectrochemistry and Bioenergetics, *2*, 69—78 (1975) and *6*, 393—403 (1979)). However, it is not easy to produce such electrodes on a large scale, because the heme protein or laccase are of animal or plant origin.

It is also known that iron, cobalt and manganese phthalocyanines and porphyrins and their drivatives catalyze the electrochemical reduction of dioxygen when they are immobilized on solid electrodes. However, dioxygen is reduced to hydrogen peroxide only (in accordance with the two-electron reaction (I) (a) above) when these electrodes are used in a neutral solution. In the case of an electrode on which dimetric cobalt porphyrin is immobilized, dioxygen is reduced directly to water only in a solution of strong acid such as 1M perchloric or trichloroacetic acid (J. Electro-analytical Chemistry, *101*, 117—122 (1979) and J. American Chemical Society, *102*, 6027—6036 (1980)). In the case of an electrode on which iron, cobalt or manganese phthalocyanine or iron protoporphyrin is immobilized, dioxygen is reduced directly to water only in a strong alkaline solution, e.g. at a pH of 12 (Bioelectrochemistry and Bioenergetics, *4*, 18—29 (1977)). In electrochemical reactors or cells containing such electrodes, dioxygen is reduced directly to water so that the electrolytic solutions are diluted by the produced water.

It has been reported that dioxygen is reduced directly to water on a dropping mercury electrode having an adsorbed cytochrome $c_3$ layer. However, mercury is not practical as an electrode material because it is poisonous.

The presence of cytochrome c in various electrochemical cells is known. Thus, in Proc. Natl. Acad. Sci. U.S.A. 73(9), 2947—9 (1976), there is disclosed a cathode of carbon disposed in a reaction mixture containing cytochrome $c_3$ hydrogenase. Also, electrodes consisting of a metallic substrate having thereon a Disulfovibrio desulfuricans microorganism are known (see US—A—3403081, US—A—3226262 and FR—A—1303200), this microorganism being one from which cytochrome $c_3$ can be obtained.

According to the present invention, there is provided an electrode comprising a solid electrically conducting material at least partly coated by a coating of cytochrome, characterised in that the coating is a monolayer coating of cytochrome $c_3$ not containing any other cytochrome.

The electrode of the invention is useful as an oxygen electrode of a fuel cell, and as an enzyme electrode.

A preferred electrode of this invention comprises a solid electrically conducting electrode material on which cytochrome $c_3$ is immobilized with a surface coverage of from 20% to 100% of monolayer coverage, the electrode being electrocatalytic active in the direct four-electron reduction of dioxygen in accordance with reaction (II) above.

The electrodes of this invention have a good performance as oxygen electrodes in the direct four-electron reduction of dioxygen. The electrodes can also be used in the electrolysis of water to form oxygen and hydrogen under appropriate conditions. One of their most important properties in their catalytic activity in the direct four-electron reduction of dioxygen to water in electrolytic solutions preferably having a pH of from 4 to 10, more preferably about 7.

Cytochrome $c_3$ is a well known biological component and may be obtained, for example, by extraction from a *Desulfovibrio microorganism.* Six different cytochromes $c_3$ have been isolated and well characterised by their chemical structure. The three dimensional structure of two cytochromes $c_3$ out of the six have further been characterised by their X-ray diffraction spectra (J. Biochemistry, *87*, 1747—1756 (1980) and *89*, 1659—1662 (1981), and Nature *282*, 806—810 (1979)). It is disclosed in the literature that the four iron-porphyrin rings are exposed toward the surface of the molecule. It is believed that heme proteins with this type of structure have a good electrocatalytic activity for the reduction of dioxygen when they are immobilized on solid electrodes. On the other hand, a molecule containing iron porphyrin surrounded by a peptide chain such as cytochrome c has no or a weak catalytic activity for the reduction of dioxygen.

Examples of *Desulfovibrio* strains from which cytochrome $c_3$ is obtained include *Desulfovibrio vulgaris Miyazaki, Desulfovibrio vulgaris Hildenborough, Desulfovibrio gigas, Desulfovibrio desulfuricans Norway, Desulfovibrio desulfuricans* and *Desulfovibrio salexigens*.

The solid electrically conducting electrodes employed in this invention are, for example, metal electrodes such as gold, platinum and nickel electrodes, and carbonaceous electrodes

such as amorphous carbon and graphite electrodes.

The immobilization of cytochrome $c_3$ on the surface of the electrode can be effected, for example, simply by soaking the electrode, the surface of which has been activated, for a period of time in a phosphate buffer solution of cytochrome $c_3$ at a preferred pH of 7.0.

The method of activation of the electrode surface is not critical. Metal electrodes are activated, for example, by treating with dilute nitric acid. Carbonaceous electrodes can be activated, for example, by plasma etching in an atmosphere of argon, carbon dioxide or oxygen, or in a vacuum. These pretreatments to activate the surface of the electrode are preferred but are not imperative.

According to this invention, electrodes should be covered with cytochrome $c_3$ in the range from 20% to 100% of monolayer coverage, preferably in the range from 20% to 80%, to give a good catalytic activity. The best catalytic activity is attained when the surface of electrodes is covered in the range from 40% to 70% of monolayer coverage.

The degree of the surface coverage of the electrode by cytochrome $c_3$ is one important point for attaining a good electrocatalytic activity in the direct four-electron reduction of dioxygen to water. When the electrode surface is covered with cytochrome $c_3$ in the above-mentioned range, the efficiency of the electrode in the direct four-electron reduction of dioxygen and in the oxidation of water is very high. The degree of the surface coverage of the electrode varies with the effective surface area of the electrode, with concentration of cytochrome $c_3$ in the buffer solution, and with the duration of soaking of the electrode in the cytochrome $c_3$ solution. Thus, for example, as can be seen from Example 1, the surface coverage by cytochrome $c_3$ of a graphite electrode which has been plasma-etched in a vacuum was about 50% of monolayer coverage when the electrode was soaked in a $1\times10^{-7}$M cytochrome $c_3$ solution for half an hour; and, as can be seen from Example 2, 100% of monolayer coverage was attained when the electrode was soaked in a $1\times10^{-4}$M cytochrome $c_3$ solution for an hour. The amount of immobilized cytochrome $c_3$ on an electrode surface is easily evaluated, for example by measuring the amount of electricity required to reduce cytochrome $c_3$ from its ferric form to its ferrous form.

Both gold and platinum were found to be appropriate substrates as the electrode material.

As is evident from the Examples, dioxygen may be reduced directly to water on the electrodes, without the formation of hydrogen peroxide as an intermediate product, in a buffer solution having a pH of from 7 to 10, preferably about 7.

The present invention is not predictable from the prior art since, according to the prior art, dioxygen is reduced either to hydrogen peroxide (in neutral solution) or to water directly (but only in strongly acid or strongly alkaline solution). Further, it is to be noted that the extent of the direct four-electron reduction of dioxygen is less in the case of electrodes covered by multilayers of cytochrome $c_3$, compared with monolayer coverage or less. As noted above, it has been reported that dioxygen is reduced directly to water on a dropping mercury electrode having an adsorbed cytochrome $c_3$ layer. However, mercury is not practical as an electrode material because it is liquid and because it is poisonous.

Known catalysts such as heme proteins and laccase are not practical catalysts because a large scale production of these catalysts is difficult. On the contrary, *Desulfovibrio* can be easily found anywhere naturally and isolated therefrom, and the large scale production of *Desulfovibrio* microorganisms is very easy (see Kagaku, *51* (6) 369—376 (1981)). In other words, cytochrome $c_3$ is advantageous as a practical catalyst for the direct four-electron reduction of dioxygen. The adsorbed cytochrome $c_3$ layer is stable, and cytochrome $c_3$ is not poisonous.

Carbonaceous electrodes as a substrate are inexpensive and are easy to fabricate.

The invention is illustrated by the following Examples and Comparative Experiments.

Example 1

A graphite electrode was pretreated with a radio frequency plasma in a vacuum so that a more than 100-fold surface area was generated.

The thus treated graphite electrode (surface area: $2\times2$ cm$^2$) was soaked in a $1\times10^{-7}$M cytochrome $c_3$ buffer solution having a pH of 7 (phosphate buffer) for half an hour, whereby about 50% of the surface was covered with a monolayer of cytochrome $c_3$. The cytochrome $c_3$ has been extracted from a *Desulfovibrio vulgaris Miyazaki* strain.

Dioxygen in a phosphate buffer solution (pH 7.0) saturated with air was reduced, using the graphite electrode as the oxygen electrode. The only reduction product was water, and no hydrogen peroxide was detectable.

Example 2

Cytochrome $c_3$ was immobilized on the graphite electrode described in Example 1 by soaking the electrode in a $1\times10^{-4}$M cytochrome $c_3$ solution (pH 7.0) for an hour, whereby a monolayer was formed over 100% of the surface of the electrode.

An electrochemical reduction of dioxygen was carried out in the same way as described in Example 1. The extent of four-electron reduction of the dioxygen was 80%.

Example 3

A similar catalytic activity for the reduction of dioxygen was obtained when Example 1 was repeated using cytochrome $c_3$ extracted from a *Desulfovibrio vulgaris Hildenborough* strain instead of that extracted from the *Desulfovibrio vulgaris Miyazaki* strain.

Example 4

Monolayer coverage was attained when a gold electrode (surface area $1\times2$ $cm^2$) was soaked in a $1\times10^{-4}$M cytochrome $c_3$ solution for 10 minutes. The cytochrome $c_3$ layer was stable and could be removed only by alternately giving the electrode the hydrogen evolution and oxygen evolution potentials.

Example 5

The oxidation of water on a gold electrode results in the formation of gold oxide on the electrode. However, when the gold electrode was coated by a cytochrome $c_3$ layer, the oxidation potential of the gold was made more positive. It was found that the amount of electricity required for the oxidation of water was about 20% more than the amount required for the reduction of the gold oxide. The difference between these amounts corresponds to the evolution of oxygen.

Comparative experiment 1

The pretreated graphite electrode described in Example 1 was covered by multilayers of cytochrome $c_3$, by soaking the electrode in a $1\times10^{-4}$M cytochrome $c_3$ solution for more than 48 hours. When the electrode was used for the reduction of dioxygen, the extent of four-electron reduction of the dioxygen was 30%.

Comparative experiment 2

The extent of four-electron reduction of dioxygen was less than 20% when using the same pretreated graphite electrode as described in Example 1, but covered by a monolayer consisting 50% of cytochrome $c_3$ and 50% of cytochrome c.

It is apparent from the foregoing Examples and Comparative Experiments that the role of cytochrome $c_3$ is remarkable as a catalyst for the four-electron reduction of dioxygen when the cytochrome c is immobilized on the surface of various solid electrically conducting materials.

**Claims**

1. An electrode comprising a solid electrically conducting material at least partly coated by a coating of cytochrome, characterised in that the coating is a monolayer coating of cytochrome $c_3$ not containing any other cytochrome.

2. An electrode as claimed in claim 1, wherein the extent of monolayer coverage is from 20 to 100%.

3. An electrode as claimed in claim 2, wherein the extent of monolayer coverage is from 40 to 70%.

4. An electrode as claimed in any one of claims 1 to 3, the cytochrome $c_3$ having been obtained from a *Desulfovibrio* microorganism.

5. An electrode as claimed in any of claims 1 to 4, the electrode having been obtained by activating the surface of the solid electrically conducting material, and by soaking the activated material in a phosphate buffer solution of cytochrome $c_3$.

6. The use of an electrode as claimed in any of claims 1 to 5 as an oxygen electrode of a fuel cell.

7. The use of an electrode as claimed in any of claims 1 to 5 as an enzyme electrode.

8. A method of effecting the catalytic four-electron reduction of dioxygen directly to water, wherein said reduction is effected in an aqueous electrolytic solution having a pH of from 4 to 10 using, as an oxygen electrode, an electrode as claimed in any of claims 1 to 5.

9. A method of effecting the electrolysis of water to oxygen and hydrogen, wherein said electrolysis is effected by the use of an electrode as claimed in any of claims 1 to 5.

**Patentansprüche**

1. Elektrode aus einem festen, elektrisch leitenden Material, das mindestens teilweise mit einer Überzugsschicht von Cytochrom beschichtet ist, dadurch gekennzeichnet, daß die Überzugsschicht eine monomolekulare Überzugsschicht aus Cytochrom $C_3$ ist, die kein anderes Cytochrom enthält.

2. Elektrode nach Anspruch 1, in der die monomolekulare Überzugsschicht in einem Ausmaß von 20 bis 100% vorliegt.

3. Elektrode nach Anspruch 2, in der die monomolekulare Überzugsschicht in einem Ausmaß von 40 bis 70% vorliegt.

4. Elektrode nach einem der Ansprüche 1 bis 3, wobei das Cytochrom $C_3$ aus einem Desulfovibro-Mikroorganismus stammt.

5. Elektrode nach einem der Ansprüche 1 bis 4, erhalten durch Aktivierung der Oberfläche des festen elektrisch leitenden Materials und durch Eintauchen des aktivierten Materials in eine Phosphatpufferlösung von Cytochrom $C_3$.

6. Verwendung einer Elektrode nach einem der Ansprüche 1 bis 5 als Sauerstoffelektrode für eine Brennstoffzelle.

7. Verwendung einer Elektrode nach einem der Ansprüche 1 bis 5 als Enzymelektrode.

8. Verfahren zur Durchführung der direkten katalytischen Vier-Elektronen-Reduktion von molekularem Sauerstoff (dioxygen) zu Wasser, wobei die Reduktion in einer wässrigen Elektrolytlösung mit einem pH-Wert von 4 bis 10 durchgeführt wird und als Sauerstoffelektrode eine Elektrode nach einem der Ansprüche 1 bis 5 eingesetzt wird.

9. Verfahren zur Durchführung der Elektrolyse von Wasser zu Sauerstoff und Wasserstoff, wobei die Elektrolyse unter Verwendung einer Elektrode nach einem der Ansprüche 1 bis 5 durchgeführt wird.

**Revendications**

1. Electrode comprenant un matériau solide conducteur de l'électricité au moins partiellement recouvert d'un revêtement de cytochrome, caractérisée en ce que le revêtement est un revêtement en couche monomoléculaire de cytochrome $C_3$ ne contenant pas d'autre cytochrome.

2. Electrode selon la revendication 1, dans laquelle le degré de couverture en couche monomoléculaire est compris entre 20 et 100%.

3. Electrode selon la revendication 2, dans laquelle le degré de couverture en couche monomoléculaire est compris entre 40 et 70%.

4. Electrode selon l'une quelconque des revendications 1 à 3, dans laquelle le cytochrome $C_3$ a été obtenu à partir d'un micro-organism Desulfovibrio.

5. Electrode selon l'une quelconque des revendications 1 à 4, dans laquelle l'électrode a été obtenue en activant la surface du matériau électriquement conducteur solide, et en trempant le matériau activé dans une solution de cytochrome $C_3$ tamponée au phosphate.

6. Utiliation d'une électrode selon l'une quelconque des revendications 1 à 5 comme électrode à oxygène d'une pile à combustible.

7. Utilisation d'une électrode selon l'une quelconque des revendications 1 à 5 comme électrode enzymatique.

8. Procédé pour effectuer la réduction catalytique à quatre électrons de l'oxygène diatomique directement en eau, dans lequel ladite réduction s'effectue dans une solution électrolytique aqueuse ayant un pH de 4 à 10 en utilisant, comme électrode à oxygène, une électrode selon l'une quelconque des revendications 1 à 5.

9. Procédé pour effectuer l'électrolyse de l'eau en oxygène et hydrogène, dans lequel ladite électrolyse s'effectue à l'aide d'une électrode selon l'une quelconque des revendications 1 à 5.